# EUROPEAN PATENT APPLICATION

(11) **EP 1 197 144 A1**
(43) Date of publication of application: **17.04.2002**
(21) Application number: 00949494.9
(22) Date of filing: 21.07.2000
(51) Int. Cl.: A01K 67/027, C12N 15/12, C12N 5/06, C07K 14/71, C07K 14/47

(54) **TRANSGENIC MICE AND OVEREXPRESSION MODEL OF THE GENE NTRK3 (TRKC) BASED THEREON FOR THE STUDY AND MONITORING OF TREATMENTS OF ANXIETY, DEPRESSION AND RELATED PSYCHIATRIC DISEASES**

(30) Priority: 23.07.1999 ES 9901674
(71) Applicant: Estivill Palleja, Xavier, E-08029 Barcelona (ES)
(72) Inventor: ESTIVILL PALLEJA, Xavier, E-08029 Barcelona (ES); GRATACOS MAYORA, Mónica, E-08029 Barcelona (ES); PUJANA GENESTAR, Miguel Angel, E-08029 Barcelona (ES); FILLAT FONTS, Cristina, E-08029 Barcelona (ES); DIERSSEN SOTOS, Mara, E-08029 Barcelona (ES)
(74) Representative: Elzaburu, Alberto de
(86) International application number: ES0000267
(87) International publication number: WO0106848

(57) **Abstract**

Lines of transgenic mice have been developed with over-express the gene NTRK3 (TRKC) located in the chromosomic region 15q24-25 which is duplicated in patients suffering from anxiety. The developed transgenic mice constitute a model for the study of physiopathology and for the development of pharmacologic, genetic and behavioral treatments against anxiety, depression and related psychiatric troubles, inter alia panic troubles, agoraphobia, social phobia, simple phobia, bipolar troubles, feed troubles (anorexia, bulimia and obesity), drug addiction, etc.

## Description

### Field of the invention

The invention deals with the development of murine models that overexpress the NTRK3 gene in its different isoforms and that will allow the study of the pathophysiology and the development of pharmacological, genetic and behavioral treatments for anxiety, depression and related psychiatric disorders.

### State of the art

Panic disorder, agoraphobia, social phobia, simple phobia and other anxiety disorders affect up to 5-10% of the general population. The prevalence of depressive disorder in adults oscillates between 12 and 17%, being the most prevalent psychiatric disorder. We have detected a duplication of about 10 megabases (Mb) in chromosome 15 (15q24-25) in individuals that suffer from anxiety disorders and depression. We have observed that the duplication 15q24-25 is closely associated to panic disorder, agoraphobia, social phobia, simple phobia and major depression. The 15q24-25 duplication is generated in multiple occasions and it is found in mosaicism in most of the patients. 6-9% of the general population has the 15q24-25 duplication, which constitutes the main genetic susceptibility factor to anxiety, depression and probably other comorbid conditions, such as eating disorders (anorexia and bulimia) and drug abuse (alcoholism and drug dependence). The duplicated region contains about 50-100 genes, and the gene that codes for the neurotrophin-3 receptor, NTRK3, (also known as TRKC) is the best candidate for anxiety disorders and depression.

Anxiety disorders are neurotic alterations that include generalized anxiety, phobic disorders, panic disorders (panic attack, panic disorders and agoraphobia) and obsessive-compulsive disorders^{1,2}. The prevalence of this group of alterations is considered to be 10% in the adult population and 5% in infantile patients³⁻⁵. It is considered that there are several millions of people affected by anxiety disorders in Europe and in the United States, but the real prevalence of these alterations is still probably higher.

Panic and anxiety disorders segregate in families, such that 25-65% of the cases have a positive family history. However, the familial transmission of anxiety disorders has often been explained by common environmental factors. The risk of first degree relatives of patients with panic disorder has been reported to be between 8 and 17%, while the prevalence of panic disorders in the general population is of 3%⁴⁻⁷. Studies in twins with anxiety disorders show a concordance of 15-63% among monozygotic twins, against 0-43% among dizygotic twins⁸⁻¹⁰. The pattern of familial transmission of panic disorder is not well defined, but it has been suggested that panic disorder and agoraphobia have an autosomal dominant pattern of inheritance with incomplete penetrance¹¹. Although a major gene for panic disorder has been postulated, a multifactorial/polygenic inheritance¹² has not been excluded.

The life-prevalence of major depressive disorder in adults oscillates between 12 and 17%, being the most prevalent psychiatric disorder^{13,14}. In spite of constituting a unique diagnostic entity, the clinical characteristics of the disorder allow us to differentiate between two depression types: the endogenous one (in turn subdivisible in bipolar and unipolar due to their clinical and even genetic and biological differences) and the reactive one¹⁵.

Anxiety disorders present an important comorbidity with depressive disorders. Panic disorder is a specially frequent diagnosis in these patients, as well as generalized anxiety or social phobia^{16,17}. The studies find that 24% of patients with panic disorder have a depressive disorder, dystimia being more common than major depression¹⁸. However, the family studies on the association between anxiety and depression have given contradictory results. In some cases panic disorder and major depression are different disorders transmitted specifically and independently, while the comorbid condition does not represent a unique or different syndrome¹⁹. In other cases it seems that there is a higher prevalence of depression in relatives of patients with panic disorder^{5,19}. Several hypothesis have intended to explain this association: 1 / the association can exist by chance, since both disorders are highly prevalent, and there is a statistically higher probability that they converge in one individual; 2 / the illness predisposes to the other one; and 3 / anxiety and depression can be two different manifestations of a single illness²⁰.

We have detected interstitial duplications of chromosome 15q24-25 in patients affected of panic disorder, agoraphobia, social phobia, simple phobia, major depression and joint laxity (familial cases and non related cases). This genomic mutation is the predisposition genetic factor for the described psychiatric disorders and it is found in 6-9% of the general population. We have developed cytologic and cytogenetic methods based on fluorescent in situ hybridization for the detection of 15q24-25 duplication and its associated mosaicism. We have developed molecular methods, based on DNA or RNA analysis for the quantitative determination of genes or other sequences in the 15q24-25 region, that can be useful for the diagnosis of anxiety and related pathologies including agoraphobia, social phobia, simple phobia, panic attack, panic disorders, major depression and other associated pathologies (patent Application 09/121.546, U.S.A., Duplications of human chromosome 15q24-25 and anxiety disorders, diagnostic methods and their detection, July 23rd 1998).

The mechanism by which the duplication 15q24-25 causes anxiety disorders and other psychiatric disorders remains unknown, but it is probably through a gene dosage effect with over-expression of one or several genes of the duplicated region. Among the candidate genes of the 10 Mb duplicated region, we should consider those genes with a possible role in the physiopathology of these disorders. For the pathology of psychiatric origin they would be the genes that are expressed in structures of the central nervous system (SNC).

The need to understand the neuropathologic basis of anxiety disorders, together with the lack of specific biological models for pharmacological assays, have generated a remarkable interest in the search for anxiety models. The efforts carried out until the present time have been focused on different lines: i) models chemically induced [colecistokinin (CCK4 and CCK8), CO2, sodium lactate, noradrenergic agonists (such as yohimbine or caffeine), GABAergic antagonists (as bicuculine), or serotoninergic drugs], ii) models obtained by means of lesion or stimulation of different brain areas (tonic inhibition of the dorsomedial hypothalamus, the selective stimulation of the amygdala or the stimulation of the periaqueductal gray substance), and iii) models obtained by means of genetic selection of animal strains that show a marked emotional reactivity in specific behavioral tests. By means of these studies, different experimental models have been generated that allow an analysis of the underlying genetic factors and different strains obtained by this method are now available. Starting from the selective mating of the murine Siracusa strain in function of the avoidance response of each animal, murine lines of high or reduced anxiety have been obtained. In a similar way, in rats of the Roman strain two lines have been obtained with differential responses at a behavioral and neuroendocrine level. The strains that present a high avoidance index and that, therefore, are considered more anxious, have been denominated roman high avoidance (RHA) and the low avoidance ones, roman low avoidance (RLA)²¹. Genetic models of mice with certain anxious behavior have also been obtained trhough chemical induction by means of the employment of mutagenic agents such as nitrosurea²².

Following a different strategy, the development of murine models of over-expression of genes contained in the mutation DUP25 constitutes the most similar approach to the genetic alteration that is responsible of the anxiety disorders and depression detected in humans. The use of these models will allow new reference points for behavioral evaluation. It is necessary to check that the models obtained are able to distinguish the characteristics of the different forms in which pathological anxiety is expressed in humans: generalized anxiety, anxiety in the social interaction (social phobia) or through panic disorders (panic attack, anxiety disorder with panic disorder or agoraphobia). One of the main limitations of the existent murine models is that these disorders can be confused, in such a way that those considered anxious can share characteristics with panic disorders in all or in some of the validation criteria. In addition, it is necessary to select appropriate experimental procedures that fulfill the pharmacological criteria of homology and detect the concrete phenotype developed. The murine models obtained will contribute to the identification of genes involved in anxiety and will also allow progress in the understanding of the pathophysiology of anxiety and its relationship with depression. Also, it will constitute a valuable pharmacological tool for the design of new drugs with a specific activity profile in the treatment of anxiety disorders and depression.

### Summary of the invention

The 15q24-25 duplication is found in 6-9% of the general population and it is the main genetic susceptibility factor for anxiety and depression, a relationship also existing with comorbid disorders, which include eating disorders (anorexia and bulimia), alcoholism and drug abuse. We have developed lines of transgenic mice that over-express the NTRK3 (TRKC) gene, located on chromosome 15q24-25, which is duplicated in patients with anxiety disorders. The characterization of TgNTRK3-ki14 indicates that the neurochemical answer of the serotoninergic system in the control animal after the administration of a panicogenic agent is significantly different from the one obtained in TgNTRK3-ki14 mice. The over-expression of NTRK3 could have a direct trophic effect on the proportion of noradrenergic neurons that interact with the locus coeruleus. This would have neurochemical consequences, increasing the noradrenergic system response to punitive environmental situations or pharmacological agents. The alteration of the functionality of the noradrenergic system could determine the alteration of the neurochemical response of other neurotransmission systems related to anxiety/depression. The TgNTRK3-ki14 mouse is a model for the study of the pathophysiology and development of pharmacological, genetic and behavioral treatments of anxiety, depression and related psychiatric disorders.

### Detailed description of the invention

The 15q24-25 duplication is involved in familial cases of several anxiety disorders and depression. This confirms the genetic and hereditary nature of these alterations and defines a common biological basis of susceptibility for these pathologies (panic disorder, agoraphobia, simple phobia, social phobia and depression), but also suggests its involvement in related illnesses such as eating disorders and drug abuse. The penetrance of this genetic alteration is not complete and the mutation is present in 6-9% of the general population. This incomplete penetrance of the 15q24-25 mutation is in agreement with the estimation of the penetrance of a predisposing allele for panic disorder under a dominant autosomic model.

The different types of 15q24-25 duplications occur in a 10 Mb region of chromosome 15, between markers D15S739 and D15S930, thus suggesting a common mutational mechanism. This mutation originates and is lost by means of unequal recombination between sister chromatids during mitosis or, more probably, due to intrachromosomic rearrangements during mitosis. A common characteristic of the 15q24-25 duplication is the occurrence of conversions among different types of duplications and between duplicated and non-duplicated chromosomes.

### NTRK3 and anxiety disorders

Among the duplicated genes in 15q24-25 the best candidate to explain anxiety disorders based on its over-expression is the gene that codes for the neurotrophin-3 receptor, NTRK3, (also known as TRKC)^{23,24}. Although neurotrophic factors are important in neurodegenerative disorders, they are also involved in neuronal plasticity, and can induce changes in the memory and learning associated connections²⁵. It has also been reported that antidepressant drugs modify the expression levels of neurotrophins and their receptors, especially neurotrophin-3 (NT-3)²⁶. In the CNS, NTRK3 is abundantly expressed in cortex, hypocampus, thalamus and hypothalamus. NTRK3 is indeed the only neurotrophin receptor detected in locus coeruleus (LC)²⁷. In the pathology of panic disorder there is a significant increment of the noradrenergic central function, because the norepinefrine system (NE) integrates and coordinates the response to fear stimuli. The LC is the main nucleus that contains NE in the SNC and plays an important role in attention and in the response to stress stimuli²⁸. The specific expression of NTRK3 in noradrenergic neurons and its localization in the duplicated region in patients with anxiety disorders, can cause its over-expression, and highlights NTRK3 as an excellent candidate for this disorders. The over-expression of NTRK3 in the LC can induce an excessive trophic effect in the NE neurons. Because the main source of NE in the brain is the LC, the final consequence of the over-expression of NTRK3 would be hyperactivity and increment of effectiveness of the NE synapsis, resulting in a dysregulation of the NE response. This higher effectiveness of the NE synapsis would diminish the emotional threshold of the individual's excitement and would alter the regulation of the fear and alarm system.

### Generation of TgNTRK3-ki14 and TqNTRK3 transgenic mice

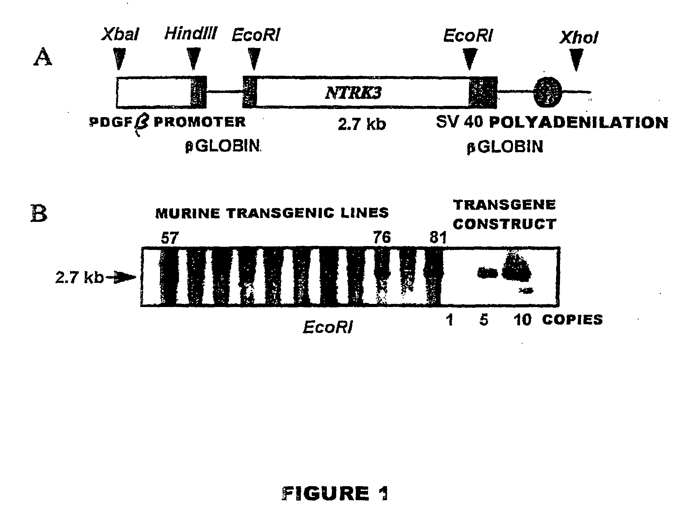

For the generation of transgenic mice we proceeded to the microinjection of the human cDNA of NTRK3-ki14 and TgNTRK3, both fused to the promoter of the PDGFB gene, according to the described methodology. This promoter was chosen since it has been demonstrated that it is able to direct the expression of the fused gene in a specific way to brain²⁹. The analysis by Southern blot allowed us to identify three independent lines of transgenic mice for the construction PDGFB-βglobin-NTRK3-kil4 (57, 76 and 81) that contained 3, 2 and 1 copy of the integrated transgene respectively (Fig 1). A line for the construction TgNTRK3 has been obtained that contains approximately 5 copies of the transgene.

### Analysis of the transgene expression

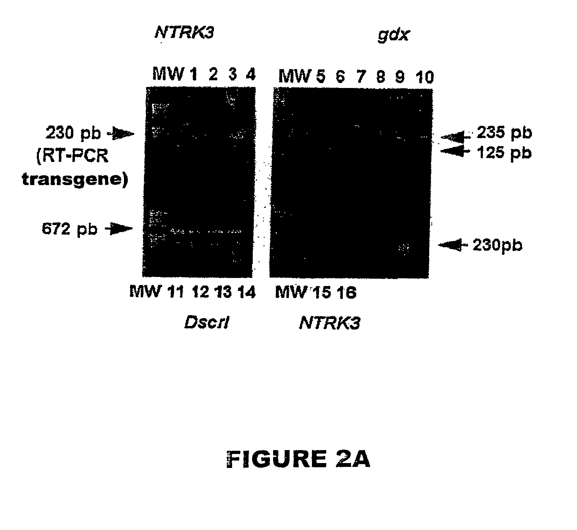

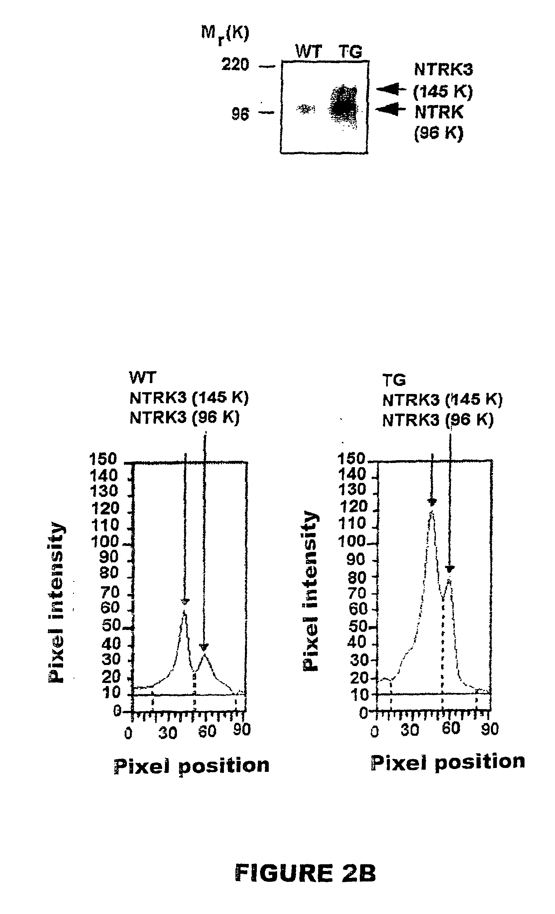

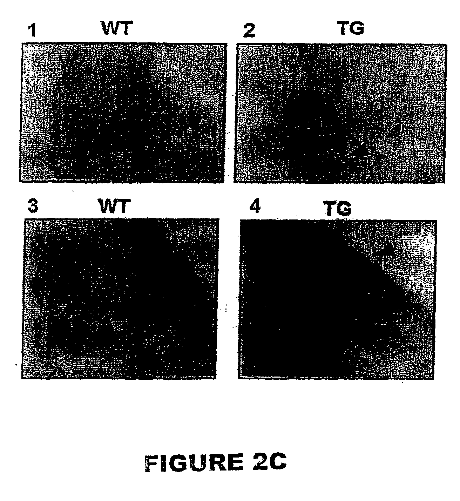

The expression studies have been carried out in the line 57 F1 generation mice of TgNTRK3-ki14. The results demonstrate that the transgene is expressed at the RNA level, determined by RT-PCR techniques, and at the protein level, as seen after the analysis by Western blot and inmunohistochemistry (Fig 2). The quantification of the expression levels has allowed us to estimate that the protein level in TgNTRK3-ki14 mice is twice that of the control animals.

### Increase in the proportion of noradrenergic neurons in the LC of TgNTRK3-ki14

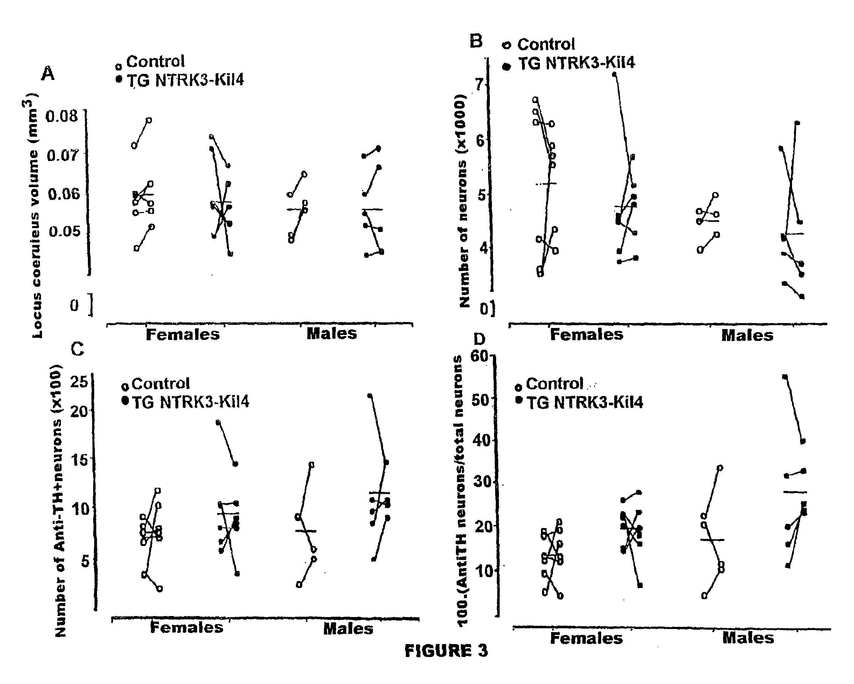

The estimates of the volumes were obtained separately for control males and females vs transgenic mice. No significant differences were observed in any experimental groups (Fig 3A).

The quantification of the neuronal number in the LC did not show significant differences among groups, although a non-significant tendency to present a higher cellular density was detected in females vs males, both in the control and TgNTRK3-ki14 groups (Fig 3B). The quantification of the neuronal population showing positive immunoreactivity toward the tyrosine hydroxylase antibody, considered as a marker for catecholaminergic neurons, did not show any significant difference in the total number of neurons. However, both male and female TgNTRK3-ki14 mice showed a non-significant tendency to present an increase in anti-TH stained neurons vs. the respective control mice (Fig 3C). These tendencies determined that the proportion of noradrenergic neurons vs. the total neuronal number in LC (100 anti-TH neuronal number/total number of neurons) was increased in TgNTRK3-ki14 vs. control mice. This increment was statistically significant in TgNTRK3-ki14 females, whereas it barely reached statistic significance in males due to the reduced number of mice in this experimental group (Fig 3D).

### Differential serotonergic response in the frontal cortex of TgNTRK3-ki14 mice after intraperitoneal injection of sodium lactate, a panicogenic agent

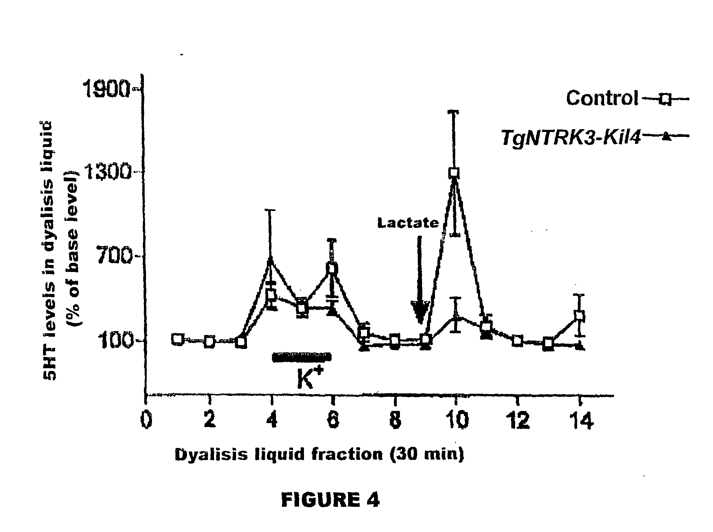

Extracellular serotonin levels were determined using in vivo microdialysis in the prefrontal cortex of control and TgNTRK3-ki14 mice. No significant differences were observed in basal serotonin levels in control and transgenic mice. Samples corresponding to basal levels of serotonin were taken for 1.5 hours. Thereafter potassium chloride (100 mM, 30 min; 1 mL/min) was infused through the microdialysis cannulae. In this stimulation condition, a significant increase in the presynaptic release of serotonin (300-400%) was obtained, that did not differ between the control and transgenic mice (Fig 4). These data indicate that the serotoninergic system in TgNTRK3-ki14 mice has no alterations in the neurotransmitter release mechanisms, thus suggesting that this neurotransmitter system has not been affected by the overexpression of TRKC-ki14. Initial experiments showed that intraperitoneal injection of sodium lactate at low doses (0.25 mEq/kg) produced a small increase in extracellular concentration of serotonin (125%) in the first 30 minutes after injection in control mice that was not observed in transgenic animals. Higher doses of sodium lactate (2.5 mEq/kg), with demonstrated panicogenic effect in rats³⁰, produced an important increase (1200 %) of extracellular serotonin concentration in control mice, that was not present in transgenic mice, where the increase attained a 300 % vs. basal values; the differences between groups being highly significant (p<0.001).

In summary the invention consists in the generation of a murine model of NTRK3 which characterization has revealed some facts that validate this model as a putative model for anxiety and depression, and probably with the related psychiatric disorders. The results indicate that the neurochemical response of the serotoninergic system that has been involved in anxiety and depression, in the presence of a panicogenic agent is significantly different in control and TgNTRK3-ki14 mice. This effect is not due to an alteration of the functionality of the serotoninergic system, but is probably attained through an indirect mechanism or pathway. The stereological studies, however indicate that over-expression of NTRK3 could have a direct neurotrophic effect on the noradrenergic neuronal population of the Locus coeruleus. This would have a neurochemical impact on the noradrenergic system, increasing the response capacity of this neurochemical system in environmental aversive situation or in response to pharmacological agents. The alteration of the functionality of the noradrenergic system could provoke the alteration in the neurochemical response of other neurotransmitter systems related to anxiety and depression.

### Methodology used in invention implementation

### Generation of transgenic mice

### 1. cDNA identification of NTRK3-ki14 and NTRK3

The human cDNA that corresponds to the isoform of NTRK3 gene with a 14 aa insertion in the extracellular protein domain (pNIJ-3)³¹ was kindly provided by Dr. Dionisio Martín Zanca (University of Salamanca) and was used for the construction of the NTRK3-ki14. To obtain the human cDNA that corresponds to the 14 aa deletion isoform, a primer with the NspHI restriction site (5'CTTCGGCATGTCCAGAGATGTCTAC3') and a primer in the 3' region of cDNA, immediately after the stop codon, with an EcoRI restriction site (5'ATACTTAAGTACTGGTGGTCGGTGG3'), were used to amplify by PCR using a brain RT as the template. The plasmid pNIJ3 was digested with the restriction enzymes NspHI and EcoRI and linked to the newly obtained fragment to obtain a new plasmid free of the insertion that was used for the design of the NTRK3 construct.

### 2. Construct design of the PDGFB-ki14 and PDGFB-NTRK3

Two independent constructs were performed for the human isoforms NTRK3-ki14 and NTRK3. The promotor-regulatory region of PDGFB gene was fused to the cDNAof the NTRK3-ki14 and the cDNA of NTRK3. With the aim of stabilizing the generated transcripts, the second intron of the β-globin gene was introduced in the 5' region of the NTRK3-ki14 and NTRK3 genes, and the polyadenylation signal of the SV-40 virus was inserted in the 3' region. The different stages of the cloning process were carried out in the Pgem-7 plasmid. Sequencing using different primers of the promoter, intronic and encoding regions ensured the integrity of the sequence. Once obtained, both constructs, PDGFB-NTRK3-ki14 and PDGFB-NTRK3, were extracted from the complete plasmid by digestion with XbaI and XhoI and subsequent electroelution. The DNA was further purified and prepared at two different concentrations (2 and 4 ng/ml) ready for microinjection.

### 3. Microinjection in fertilized oocytes

Manipulation of mouse embryos and microinjection were performed using standard protocols. Embryos at the unicellular stage were obtained from the oviducts of female B6SJL F1. The embryos were microinjected with the chimeric genes PDGFB-NTRK3-ki14 or PDGFB-NTRK3. The embryos surviving the microinjection process were transferred to CD1 pseudopregnant females and at three weeks of age, after the weaning period the presence of the transgene was analyzed.

### 4. Southern blot analysis of transgenic mice

Southern blot analysis was used in order to identify transgenic mice. Genomic DNA was obtained from tail biopsies. 10 µg of DNA was digested with EcoRI, the fragment generated was separated in agarose gels. DNA was then transferred to nitro-cellulose membranes and was hybridized with a probe, corresponding to the complete human NTRK3 cDNA. The probes were radioactive labeled with ³²P by random priming method³². The hybridization was performed at 65° C in Denhardts-SSC buffer and was washed in 0.1x SSC, 0.1x SDS during 15 min. The filters were exposed to a Xray film (Curix, Agfa) overnight at -80° C. The analysis of the resulting band allowed us to identify a 2.7 kb fragment corresponding to the transgene.

### Expression of NTRK3-ki14 and NTRK3 in TgNTRK3-ki14/TgNTRK3 mice

### 1. Number of copies of the transgene

In order to determine the copy number inserted in each transgenic line, different amounts of construct DNA corresponding to 1, 5, and 10 copies of the construct present in 10 µg of genomic DNA were digested with EcoRI. The hybridization signal doses were determined in the autoradiographies by quantification of the band's intensity with a densitometer (Phoretix, International, Newcastle, UK).

### 2. Analysis of the levels of expression of the transgenes by RT-PCR

For the analysis of the expression of the transgenes, total RNA was obtained from brains of control and transgenic mice. The RNA was isolated using the Rneasy Mini Kit (Qiagen). The different total RNA were reverse transcribed using the GeneAmp RNA Superscript protocol (Gibco-BRL). The primers used for the analysis of expression of the transgene in the RNA reverse transcribed were designed avoiding both the exon and the zones of homology between the mice and human NTRK3 sequences. Hybridization of the human and mice sequences using BLAST2 sequences (NCBI) program achieved the identification of the low homology regions. The primers were: NTRK3hum/mou-F 5' - TGTTTGACGAAGTGAGTCCC- 3' and NTRK3hum/mou-R 5' - TCCAGTGACGAGGGCGTG- 3'. To identify the possible contamination of genomic DNA in the reverse transcription, two strategies were employed: 1. Samples were treated with DNAse RNAse Free (Boehringer Manheim), and 2. A GdX fragment was amplified with the primers gdx-F 5'-GGCAGCCCCCTGATCTCCAAAGTCCTGG-3' and gdx-R 5'-AACGTTCGATGTCATCCAGTGTTA-3' giving rise to fragments of different size if amplified from RNA (125 bp) or DNA (235 bp).

### 3. Western blot analysis and quantification of NTRK3-ki14 expression

Total lysates of transgenic and control mice were obtained in lysis buffer RIPA (PBS 1% Tx100, 0.1% SDS, 5 nM EDTA, 2.5 U/ml aprotinine and 1 nM PMSF). After clearance of the lysates by centrifugation at 1000x g, 15 min and at 100,000 x g for 30 min, protein quantification was performed following the RCA Protein Assay Reagent (Pierce) protocol. Equivalent quantities of lysates (1 to 2 mg) were immunoprecipitated with anti-panTrk (5 µg) (Promega) and protein-A sepharose (Pharmacia). The immunocomplexes were then recovered by centrifugation and were resuspended in electrophoresis buffer SDS 7% polyacrilamide and subsequent elecrophoretic transference to a Hybond-P membrane (Amersham). To perform the western blot the primary antibody anti-panTrk (1:1000) (Promega) and the secondary peroxidase-conjugated anti-chicken IgY (1:1000) were used. The protein detection was performed following the ECL system protocol (Amersham). The quantification of the obtained bands was done by densitometric analysis (phoretix).

### 4. Analysis of the expression of NTRK1-ki14 by immunohistochemistry

For the immunohistochemical analysis mice were perfused with a 4% paraformaldehide solution, dissected and fixed during 24 h. 50 µm cerebral sections were obtained that were subsequently washed with PBS buffer and thereafter maintained in glycerol. A previous blockage of the endogenous peroxidases was performed with a PBS, 3% oxygenated water, and 10% methanol solution. The blockage of the unspecific binding was ensured with the addition of a solution containing PBS, 0.2% Tx100, 0.2% gelatine, 0.2% azide, 0.2% glycine and 0.2% lysine. For the detection of the proteins TrkC (Ntrk3) and TrkC-ki14 (NtrkC-ki14) a polyclonal rabbit antiTrkC antibody (1:100) (Santa Cruz Biotechnology, Inc.) was used. The incubation of the antibody was in 10% normal serum. Development was with a PBS, 0.05% DAB and 0.1% oxygenated water solution.

### Functional studies in TgNTRK3-ki14 and TgNTRK3 mice

### 1. Stereological studies in TgNTRK3-ki14 mice

Stereology allows the non-biased quantitative study of the number, the extension and volume of biological structures. The main advantage of this method is its efficiency such that with a reduced number of data a highly precise estimation is obtained. For the stereological studies, 11 TgNTRK3-ki14 and 9 control mice (20-25 gr) were used. The brains were dissected and included overnight in a fixation solution (4% paraformaldehide, 2% picric acid in phosphate buffer, 0.1M, pH=7.3) at 4° C. Thereafter, the fixation solution was substituted by sacarose (30%) and finally by an antifreezing solution. The brains were stored at -20° C until their use. Before use, brains were again submerged in 30% sacharose and serial 50 mm sections were obtained in a criostate (2800 Frigocut, Reichert-Jung). The tissue sections were mounted on gelatinized slides and were maintained in cresyl violet solution for 2 hours at 40°C. After completion of the staining, the section were dehydrated by means of increasing concentrations of alcohol and were mounted with DPX (Fluka). The estimation of the volume was performed by the method of Cavlieri³³ that allows a non-biased estimation of the volume of any object by the sum of the areas of parallel sections of the object, separated by a known distance. The obligatory condition is that the first section must be randomly selected. The measure of the area of each section is estimated by the used of a template, in which each of the drawn points has an associated area. The measures were taken by means of the CAST-GRID software adapted to an OLYPMUS microscope. For the cellular count in 50 mm sections, the optic dissector method was used, that consists in the systematic use of a sampling window, the dissector, of dimensions selected by the experimenter. In each preparation, the area of interest is selected using the smallest objective (2.5x) making random subdivisions at a specific size selected by the experimenter in the XY axes. In each of the subdivisions the dissector determines the area where the neurons will be counted. It is estimated that we are at the surface level when the first structure of the area is focused. A security zone of 3 mm was considered to ensure that all neurons are from the selected section. All those neurons which nucleoli in focus under 3 mm and before the height determined by the dissector (10 mm) are included. For the count the objective of immersion 100x is used. In the present analysis the volume, total number of neurons, and anti-TH (tyrosine hydroxylase) positive neurons in the LC were studied.

### 2. Microdialysis experiments in vivo in TgNTRK3-ki14 mice

In the microdialysis experiments 6 control and 7 TgNTRK3-ki14 mice were used (20-25 gr). For the implantation of the dialysis probes, the mice were anaesthetized with Avertin (Tribromoetanol/Tertamilalcohol 1.6/1 p/p; 0.1 mL/10 g) administered intraperitoneally. After the anaesthesia was achieved, mice were immobilized in a stereotaxia apparatus and the microdialysis probes (Cuprophane, cut-off = 6000 Dalton; the membrane is 2 MM. length and 0.24 external diameter) were implanted inside the cingular cortex (stereotaxic co-ordinates with respect to Bregma: anterior: 1.5 mm; lateral: -0.04 mm; Paxinos). The position of the probe was histologically verified. After surgery, the animals were returned to their cages with food and water ad libitum. 24 hours later the animals were transferred to transparent dyalization cages provided with balanced supporting arm to avoid the torsion of the perfusion cannulae. At the beginning of the experiment the dyalization probe was connected to an infusion bomb and Krebs solution was infunded at 1 µL/min. After the equilibration period of one hour, dialyzed samples were taken each 20 min. Samples were taken in basal conditions, after stimulation with KCl 100 mM and after the intraperitoneal administration of sodium lactate (0.5-2.5 mEq/kg), a panicogenic agent³⁰. The dialyzates were analyzed to determine the content of serotonin by means of HPLC with electrochemical detection. The monoamines were separated in microborum columns of reverse phase (C-18; 5 mm, 1 x 150 mm), using a mobile phase of citrate-phosphate buffer 0.03M, with octanoic acid 2.1 mM; EDTA 0.1 mM and 17% metanol (pH = 3.6, at a rate of 90 µL/min). The detection is performed with a 3 mm electrode at + 0.85 v. The injection volume was 5 µL. For the statistic comparisons the data are presented as mean ± SEM. Analysis was with ANOVA using Bonferroni's analysis as the post hoc test.

### References

1) American Psychiatric Association. Diagnostic and Statistical Manual of Mental Disorders (DSM-III-R). 3rd ed, revised. The American Psychiatric Association, Washington DC, (1987).
2) American Psychiatric Association, Diagnostic and Statistical Manual of Mental Disorders (DSM-IV). 4th ed. The American Psychiatric Association, Washington DC, (1994).
3) Klerman, G.L., Weissman, M.M., Quellette, R., Johnson, J. and Greenwald, S. Panic attack in the community. Social morbidity and health care utilizations. JAMA 13, 742-746 (1992).
4) Eaton, W.W., Kessler, R.C., Wittchen, H.U. and Magee, W.J. Panic and panic disorder in the United States. Am. J. Psych. 151, 413-420 (1994).
5) Weissman, M.M. et al. The relationship between panic disorder and major depression. Arch. Gen. Psychiatry 50, 767-780 (1993).
6) Crowe, R.R., Noyes, R., Pauls, D.L. and Slymen D. A family study of panic disorder. Arch. Gen. Psychiatry 40, 1065-1069 (1983).
7) Weissman, M.M., Bland, R.C., Canino, G.J., et al. The cross-national epidemiology of panic disorder. Arch. Gen. Psychiatry 54, 305-309 (1997).
8) Andrews, G., Stewart, G., Allen, R. and Henderson, A.S. The genetics of six neurotic disorders: a twin study. J. Affec. Dis. 19, 23-29 (1990).
9) Kendler, K.S., Neale, M.C., Kessler, R.C., Heath, A.C. and Eaves, L.J. Panic disorder in women: a population-based twin study. Psychol. Med. 23, 397-406 (1993).
10) Perna, G., Caldirola, D., Arancio, C. and Bellodi, L. Panic attacks: a twin study. Psychiatr. Res. 66, 69-71 (1997).
11) Vieland, V.J., Hodge, S.E., Lish, J.D., Adams, P. and Weissman, M.M. Segregation analysis of panic disorder. Psychiatr. Gene. 3, 63-71 (1993).
12) Knowles, J.A., Vieland, V.J., Fyer, A.J. et al. Panic disorder is unlikely to be a homogeneous autosomal dominant didorder: results of a genomic-wide genetic screen. Am. J. Hum. Genet. 57S4, A1123 (1995).
13) Hirschfeld R, and Cross CH. Epidemiology of affective disorders. Arch. Gen. Psychiatry 39, 35-46 (1982).
14) Kessler RC, McGonagle KA, Zhao S et al. Lifetime and 12-month prevalence of DSM-III psychiatric disorders in the United States: results from the National Comorbidity Survey. Arch. Gen. Psychiatry 51, 8-19 (1994).
15) Kendell RE. The classification of depressions: a review of contemporary confusion. Br. J. Psychiatry 129, 15-28 (1976).
16) Sanderson W, Beck A and Beck J. Syndrome comorbidity in patients with major depression or dysthymia: prevalence and temporal relationships. Am. J. Psychiatry 147, 1025-1028 (1990).
17) Davies F, Norman R, Cortese L and Malla A. The relationship between types of anxiety and depression. J. Nerv. Ment. Dis. 183, 31-35 (1995).
18) Wetzler S, and Sanderson W. (1995). Comorbidity of panic disorder. En Panic disorder: clinical, biological and treatment aspects. Ed. Wiley, New York, pp.80-98.
19) Maier W, Lichtermann D, Minges J, Oehrlein A and Franke P. A controlled family study in panic disorder. J. Psychiatr. Res. 27, 79-87 (1993).
20) Frances A, Manning D, Marin D et al. Relationship of anxiety and depression. Psychopharmacology 106, 82-86 (1992).
21) Meerlo P, Overkamp GJ and Koolhaas JM. Behavoiral and physiological consequences of single social defeat in Roman high and low-avoidance rats. Psychoneuroendocrinology 22, 155-168. 1997
22) Brown, S. and Peters, J. Combining mutagenesis and genomics in the mouse - closing the phenotype gap. Trends Genet. 12, 433-32 (1996).
23) Lamballe, F., Klein, R., and Barbacid, M. TrkC, a new member of the trk family of tyrosine protein kinases, is a receptor for neurotrophin-3. Cell 66, 967-979 (1991).
24) Mcgregor, L.M., Baylin, S.B., Griffin, C.A., Hawkins, A.L. and Nelkin, B.D. Molecular cloning of the cDNA for human TrkC (NTRK3), chromosomal assignment, and evidence for a splice variant. Genomics 22, 267-272 (1994).
25) Kang, H. and Shuman, E. Long-lasting neurotrophin induced enhancement of synaptic transmission in the adult hippocampus. Sicence 267, 1658-1662 (1995).
26) Smith, M.A. et al. Stress and antidepressants differentially regulate neurotrophin 3 mRNA expression in the locus coeruleus. Proc. Natl. Acad. Sci. USA 92, 8788-8792 (1995).
27) Merlio, J.P., Ernfors, P, Jaber, M. and Persson, H. Molecular cloning of rat trkC and distribution of cells expressing messenger RNAs for members of the trk family in the rat central nervous system. Neuroscience 51, 513-532 (1992).
28) Foote, S.L., Bloom , F.E., and Aston-Jones, G. Nucleus locus ceruleus: new evidence of anatomical and physiological specificity. Physiol. Rev. 63, 844-914 (1983).
29) Sasahara M, Fries JWU, raines EW, Gown AM, Westrum LE, Frosch MP, Bonthron DT, Ross R, and Collins T. PDGF B-Chain in neurons of the Central Nervous System, posterior pituitary, and in a transgenic model. Cell 64, 217-227 (1991).
30) Den Boer JA, Westenberg HG, Klompakers AA, and van Lint LE. Behavioral biochemical and neuroendocrine concomitants of lactate-induced panic anxiety. Biol. Psychiatry 26, 612-622 (1989).
31) Shelton DL, Sutherland J, Gripp J, Camerato T, Armanini MP, Philips HS, Carroll K, Spencer SD, and Levinson AD. Human trks: molecular cloning, tissue distribution and expression of extracellular domain inmunoadhesins. J. Neurosci. 15, 477-491 (1995).
32) Sambrok J, Fritsch EF and Maniatis T. Molecular cloning. A laboratory manual. Cold Spring Harbor Laboratory Press. Cold Spring Harbor, New York (1989).
33) Mayhew TM and Gundersen JG. (1996). If you assume, you can make an ass out of you and me: a decade of the director for stereological counting of particles in 3D space. J. Anat. 188, 1-15.

### Figure legends

Figure 1
   A) Structure of the construct used to obtain the transgenic mice TgNTRK3-ki14 and TgNTRK3. B) EcoRI restriction enzyme analysis of genomic DNA from three transgenic lines (57, 76 and 81) and quantification of the number of inserted copies, 1, 5 and 10.
Figure 2
   A) Detection of the expression of NTRK3-ki14 in the murine line 57. Lane 1: RT-PCR with primers trkC hum/mou F/R from total RNA obtained from brain of a wildtype mouse (WT); lane 2, of a TgNTRK3-ki14 (TG) mouse; lane 3, RT-PCR with primers trkC hum/mou F/R from total RNA obtained from brain of a WT mouse and treated with DNAse RNAse free (Boheringer Manheim); lane 4, of TG mouse; lanes 5-8, controls of genomic contamination in the RT-PCR reactions using primers of the murine gdx gene, which allowed us to discriminate between RNA (125 bp) and DNA (235 bp) amplification; lanes 5 and 6, of WT and TG respectively, without treatment with DNAse RNAse free; lanes 7 and 8, of WT and TG mice respectively, with treatment with DNAse RNAse free; lanes 9 and 10, controls of genomic murine DNA; lanes 11-14, control of RT-PCR with specific primers of the murine gene dscr1 (672 bp); lane 15, control trkC hum/mou F/R from the construct; lane 16, control of primers trkC hum/mou F/R; MW, molecular weight marker. B) Western blot of immunoprecipitate with anti-pantrk (Promega) from total proteins of WT and TG mouse brains from line 57, and quantification by densitometry. C) Immunohistochemistry of slices from WT and TG mice of line 57. 1, hippocampus of WT mouse; 2, hippocampus of TG mouse; 3, cerebral cortex of WT mouse; 4, cerebral cortex of TG mouse.
Figure 3
   Stereologic analysis in TgNTRK3-ki14 and controls. A, measure of the volume of the LC. B, quantification of the number of neurons in the LC. C and D, quantification of the antitirosine hydroxylase immunorreactive neuronal population.
Figure 4
   Serotoninergic response against the sodium lactate in TgNTRK3-ki14 and control mice. K+, indicates infusion of KCl. The arrow indicates the infusion of sodium lactate.

## Claims

1. TgNTRK3 transgenic mice that over-express the NTRK3 gene complete coding sequence under the control of the PDGFB gene promoter.

2. TgNTRK3-kil4 transgenic mice that over-express the NTRK3 gene isoform with an insertion of 14 amino acids in the NTRK3-kil4 kinase domain under the control of the PDGFB gene promoter.

3. Use of the transgenic mice of claim 1 as model for the study of the pathophysiology of anxiety disorders such as panic disorder, agoraphobia, social phobia, simple phobia and generalised anxiety.

4. Use of the transgenic mice of claim 2 as model for the study of the pathophysiology of anxiety disorders such as panic disorder, agoraphobia, social phobia, simple phobia and generalised anxiety.

5. Use of the TgNTRK3 and/or TgNTRK3-kil4 transgenic mice that over-express the NTRK3 or NTRK3-kil4 isoforms of the NTRK3 gene, as described in claims 1 and 2, as models for the study of the pathophysiology of depression disorders, such as major depression and bipolar disorder.

6. Use of the TgNTRK3 and/or TgNTRK3-kil4 transgenic mice that over-express the NTRK3 or NTRK3-kil4 isoforms of the NTRK3 gene, as described in claims 1 and 2, as models for the study of the pathophysiology of anxiety-related eating disorders, such as anorexia, bulimia and obesity.

7. Use of the TgNTRK3 and/or TgNTRK3-kil4 transgenic mice that over-express the NTRK3 or NTRK3-kil4 isoforms of the NTRK3 gene, as described in claims 1 and 2, as models for the study of the pathophysiology of substance abuse disorders, such as drug addiction.

8. Use of any of the models based on the transgenic mice described in claims 1 or 2 or on any other transgenic mice that over-express the NTRK3 and/or NTRK3-kil4 genes for the identification, monitoring and study of phenotypes of anxiety, eating disorders and drug addiction induced by environment manipulation.

9. Use of any of the models based on the transgenic mice defined in claims 1 and 2 or on any other transgenic mice that over-express the NTRK3 and/or NTRK3-kil4 genes for the identification, monitoring and study of phenotypes of anxiety, depression, eating disorders and drug addiction induced by pharmacological treatment.

10. Use of the transgenic mice described in claims 1 and 2 or on any other transgenic mice that over-express the NTRK3 and/or NTRK3-kil4 genes for the monitoring of useful compounds for the pharmacological treatment of anxiety disorders, depression, eating disorders and drug addiction.

11. Use of the transgenic mice described in claims 1 and 2 or any other transgenic mice that over-express the NTRK3 and/or NTRK3-kil4 genes for the monitoring of behavioural treatments of anxiety disorders, depression, eating disorders and drug addiction.

12. Use of the transgenic mice described in claims 1 and 2 or any other transgenic mice that over-express the NTRK3 and/or NTRK3-kil4 genes for the monitoring of useful compounds for gene transfer treatments, including antisense sequences, for anxiety disorders, depression, eating disorders and drug addiction.
